# EUROPEAN PATENT SPECIFICATION

(11) **EP 2 713 995 B1**
(45) Date of publication and mention of the grant of the patent: **03.08.2016**
(21) Application number: 12724846.6
(22) Date of filing: 29.05.2012
(51) Int. Cl.: A61K 8/31, A61K 8/34, A61K 8/42, A61K 8/891, A61Q 5/00, A61Q 5/12

(54) **CLEAR HAIR CARE COMPOSITION COMPRISING BASE OIL AND HYDROPHILIC COMPONENT**
KLARE HAARPFLEGEZUSAMMENSETZUNG MIT BASISÖL UND HYDROPHILEM BESTANDTEIL
COMPOSITION TRANSPARENTE DE SOINS CAPILLAIRES COMPRENANT UNE HUILE DE BASE ET UN CONSTITUANT HYDROPHILE

(30) Priority: 03.06.2011 US 201161492843 P
(43) Date of publication of application: 09.04.2014
(73) Proprietor: The Procter & Gamble Company, Cincinnati, OH 45202 (US)
(72) Inventor: HASEGAWA, Jun, Hyogo 658-0063 (JP); UEHARA, Nobuaki, Singapore 649823 (SG); OKU, Taisuke, Hyogo 6570016 (JP)
(74) Representative: Sauvaître, Thibault Bruno
(86) International application number: PCT/US2012/039809
(87) International publication number: WO 2012/166698

(56) References cited:
- WO-A1-2010/087004
- JP-A- H02 172 905
- US-A- 824 353
- US-A1- 2003 152 539
- US-A1- 2005 152 539
- PERILLO M A ET AL: "Surface behavior of jojoba oil alone or in mixtures with soybean oil", COLLOIDS AND SURFACES. A, PHYSICACHEMICAL AND ENGINEERING ASPECTS, ELSEVIER, AMSTERDAM, NL, vol. 256, no. 2-3, 22 April 2005 (2005-04-22), pages 199-205, XP027803004, ISSN: 0927-7757 [retrieved on 2005-04-22]

## Description

### FIELD OF THE INVENTION

The present invention relates to a non-aqueous clear hair care composition comprising: a base oil consisting of an isoparaffin, a mineral oil, a volatile silicone compound, and mixtures thereof; an hydrophilic material; and a liquid fatty alcohol having 10 carbon atoms or more; wherein the weight ratio of the hydrophilic material to the liquid fatty alcohol is from 1:85 to 1:600 as defined in the claims. By the use of liquid fatty alcohol, hydrophilic materials are dissolved in the base oil and the composition has a clear product appearance.

### BACKGROUND OF THE INVENTION

Human hair becomes soiled due to its contact with the surrounding environment and from the sebum secreted by the scalp. The soiling of hair causes it to have a dirty feel and an unattractive appearance. The soiling of the hair necessitates shampooing with frequent regularity.

Shampooing cleans the hair by removing excess soil and sebum. However, shampooing can leave the hair in a wet, tangled, and generally unmanageable state. Once the hair dries, it is often left in a dry, rough, lusterless, or frizzy condition due to removal of the hair's natural oils and other natural conditioning and moisturizing components. The hair can further be left with increased levels of static upon drying, which can interfere with combing and result in a condition commonly referred to as "fly-away hair," or contribute to an undesirable phenomenon of "split ends." Further, chemical treatments, such as perming, bleaching, or coloring hair, can also damage hair and leave it dry, rough, lusterless, and damaged.

A variety of approaches have been developed to condition the hair. A common method of providing conditioning benefits to the hair is through the use of application of hair conditioning composition. Conditioning formulations can be in the form of rinse-off products or leave-on products, and can be in the form of an emulsion, cream, gel, spray, mousse, oil, liquid and serum.

For leave-on products especially those in a non-aqueous liquid form (such as oil, liquid and serum) comprising a larger amount of oily compounds, there was a need for such products to include hydrophilic materials such as panthenol. For example, US 2003/0017179 relates to, according to Claim 1, a liquid oil-based cosmetic comprising a main ingredient of at least one kind of animal and vegetable oils, 0.01-6.0wt% of panthenol, 0.4-10wt% of a glycol having an average molecular weight of 90-400, and 5-40% of monohydric alcohol having an average molecular weight of 110-250. This US publication discloses in Example No. 10 a composition comprising 2.0% panthenol, 8.0% polyethylene glycol 400, 10% 1-dodecanol, 10% isopropyl palmitate, and 61.7% safflower oil. This US publication discloses in Example No. 13 a composition comprising 2.0% panthenol, 8.0% dipropylene glycol 400, 30% 1-dodecanol, 10% isopropyl palmitate, and 41.7% squalane. The US publication further discloses in Comparative Examples, compositions comprising 2.0% panthenol, 30% oleyl alcohol, 10% isopropyl palmitate, and 41.7% squalane.

Another example is Australian patent application publication No. AU199642210. This AU publication relates to, according to Claim 1, a hair and scalp treating composition comprising: 0.01-99.99wt% of an oil selected from the group consisting of silicone oils, hydrocarbon oils, fluorocarbon oils, vegetable oils, mineral oils, and mixtures thereof; 0.0001-50wt% of a polar active ingredient selected from water soluble B complex vitamins; 0.001-50wt% of a surface active agent selected from the group consisting of anionic surfactants, cationic surfactants, amphoteric surfactants, zwitterionic surfactants, nonionic surfactants, and mixtures thereof. The AU publication discloses in Example IV a hair oil composition (water-in-oil emulsion) comprising a total 60% mineral oil, q.s. to 100% grandnut oil and 0.001% panthenol, 0.009% panthenyl ethyl ether, and 2% laureth-3.

Furthermore, WO2010/0870 discloses hair oils comprising a dibasic ester compound and one or more oils selected from the group consisting of a volatile oil, an ester oil, a hydrocarbon oil, an animal or plant oil and a silicone oil. The oily hair cosmetic aims to improve damaged hair feeling.

However, there remains a need for such products which provide reduced greasy feel, while solubilizing hydrophilic components such as panthenol.

None of the existing art provides all of the advantages and benefits of the present invention.

### SUMMARY OF THE INVENTION

The present invention is directed to a non-aqueous clear hair care composition comprising by weight:
from 61% to 99.9% of a base oil consisting of: an isoparaffin, a mineral oil, a volatile silicone compound, and mixtures thereof;
from 0.0005% to 0.1 % of an hydrophilic material;
from 0.035% to 15% of a liquid fatty alcohol having 10 carbon atoms or more;
wherein the weight ratio of the hydrophilic material to the liquid fatty alcohol is from 1:85 to 1. 600; and 1 % or less of water by weight of the composition.

The hair care compositions of the present invention provide reduced greasy feel, while solubilizing hydrophilic components such as panthenol.

These and other features, aspects, and advantages of the present invention will become better understood from a reading of the following description, and appended claims.

### DETAILED DESCRIPTION OF THE INVENTION

While the specification concludes with claims particularly pointing out and distinctly claiming the invention, it is believed that the present invention will be better understood from the following description.

Herein, "comprising" means that other steps and other ingredients which do not affect the end result can be added. This term encompasses the terms "consisting of" and "consisting essentially of".

All percentages, parts and ratios are based upon the total weight of the compositions of the present invention, unless otherwise specified. All such weights as they pertain to listed ingredients are based on the active level and, therefore, do not include carriers or by-products that may be included in commercially available materials.

Herein, "mixtures" is meant to include a simple combination of materials and any compounds that may result from their combination.

### COMPOSITION

The composition of the present invention is a non-aqueous clear hair care composition comprising by weight:
from 61% to 99.9% of a base oil consisting of: an isoparaffin, a mineral oil, a volatile silicone compound, and mixtures thereof;
from 0.0005% to 0.1% of an hydrophilic material;
from 0.035% to 15% of a liquid fatty alcohol having 10 carbon atoms or more;
wherein the weight ratio of the hydrophilic material to the liquid fatty alcohol is from 1:85 to 1:600; and 1% or less or water by weight of the composition.

It has been found by the inventor of the present invention that, when the composition comprises the above base oil at the above level, the composition provides reduced sticky/greasy feel, compared to other base oils such as those containing a larger amount of vegetable oils including safflower oil. However, it has been surprisingly found by the inventor of the present invention that, when the composition comprises the above base oil at the above level, it becomes difficult to dissolve hydrophilic materials such as panthenol in the composition.

The inventors of the present invention have found that, by the addition of specific liquid alcohol at a specific ratio, hydrophilic materials are dissolved in the composition comprising the above base oil, and the composition has a clear product appearance.

### Non aqueous composition

The composition of the present invention is a non-aqueous composition. Non-aqueous composition herein means that the composition is substantially free of water. In the present invention, "the composition being substantially free of water" means that the level of water, is 1% or less, preferably 0.5% or less, more preferably 0.3% or less, still more preferably 0.1% or less, even more preferably 0% by weight of the composition.

### Turbidity

The composition of the present invention has a homogeneous clear product appearance, i.e., homogeneous transparent product appearance.

Preferably, the composition of the present invention has a turbidity of 2.0NTU or less, 1.5NTU or less, more preferably 1.0NTU or less, still more preferably 0.7NTU or less at 25°C. Further preferably, the composition of the present invention has such above turbidity at 25°C, even after its storage at 5 °C for at least 1 hour. The transmittances are measured by using HACH 2100N Turbidimeter.

In view of homogeneous clear product appearance, it is preferred to control the level of components which are substantially insoluble to the composition of the present invention. By "substantially insoluble" compound, what is meant is that:
the compound is substantially insoluble in the compositions at the level used; and,
when containing the compounds at the level used, the compositions are either: (i) nonhomogeneous by, for example, phase separation; or (ii) homogeneous but with a higher turbidity, i.e., turbidity of above 2.0NTU (excluding 2.0NTU), preferably above 1.5NTU (excluding 1.5NTU), more preferably above 1.0NTU (excluding 1.0NTU), still more preferably above 0.7NTU (excluding 0.7NTU) at 25°C.

Such substantially insoluble compounds further include fatty compounds including, for example: fatty alcohols having a melting point of 25°C or more, such as cetyl alcohol and stearyl alcohol; fatty acids having a melting point of 25°C or more, such as stearic acid; fatty alcohol derivatives and fatty acid derivatives such as esters and ethers thereof, which derivatives have a melting point of 25°C or more; and mixtures thereof.

When the base oil is a volatile silicone, such substantially insoluble compounds further include, fatty alcohol derivatives, fatty acids, and fatty acid derivatives, those having a lower melting point than 25°C.

More preferably, the compositions of the present invention are substantially free of such substantially insoluble compounds. In the present invention, the compositions being "substantially free" of substantially insoluble compounds means that: the composition is free of substantially insoluble compounds; or, if the composition contains substantially insoluble compounds, the level of such substantially insoluble compounds is very low. In the present invention, the level of such substantially insoluble compounds is, if included, 1.0% or less, preferably 0.5% or less, more preferably 0.3% or less, still more preferably 0.1% or less, even more preferably 0%.

### Substantially free of surfactants

Preferably, the composition of the present invention is substantially free of surfactants, in view of product clarity and/or reduced undesirable foaming of the product.

Such surfactants include: anionic surfactants; cationic surfactants; amphoteric surfactants; zwitterionic surfactants; nonionic surfactants having a HLB of 5 or more; and mixtures thereof. Such nonionic surfactants having a HLB of 5 or more includes, for example: ethers of fatty alcohols such as ethers of lauryl alcohol including Laureth-3 (having a HLB of about 8); esters of fatty alcohols such as esters of lauryl alcohols.

In the present invention, "the composition being substantially free of surfactants" means that: the composition is free of surfactants; or, if the composition contains surfactants, the level of such surfactants is very low. In the present invention, the total level of such surfactants is, if included, preferably 0.1% or less, more preferably 0.01% or less, still more preferably 0.001% or less, even more preferably 0.0005% by weight of the composition.

### BASE OIL

The composition of the present invention comprises a base oil. The base oil is included at a level by weight of the composition of, from about 61% to about 99.9%, from about 70% to about 99.5%, more preferably from about 75% to about 99.0%, still more preferably from about 80% to about 98%, still more preferably from about 85% to about 98%.

The base oil useful herein consists of an isoparaffin, a mineral oil, a volatile silicone compound, and mixtures thereof. Preferably, the base oil consists of an isoparaffin, a mineral oil, and mixtures thereof, in view of balance between reduced sticky/greasy feel and conditioned feel which could be delivered by a higher viscosity.

### Isoparaffin

The isoparaffins useful herein have a viscosity of, preferably from about 0.5mm². s⁻¹ to about 50mm² s⁻¹, more preferably from about 0.8mm²· s⁻¹ to about 40mm²· s⁻¹, still more preferably from about 1mm²· s⁻¹ to about 30mm²· s⁻¹, even more preferably from about 1.5mm²· s⁻¹ to about 20mm²· s⁻¹, further more preferably from about 1.5mm²· s⁻¹ to about 10mm²· s⁻¹ at 37.8°C. When using two or more isoparaffin, it is preferred that the mixture of isoparaffin have the above viscosity.

Isoparaffins useful herein are preferably volatile. Preferably, the volatile isoparaffin useful herein are those having from about 8 to about 20 carbon atoms, more preferably from about 8 to about 16 carbon atoms, still more preferably from about 8 to about 12 carbon atoms, even more preferably from about 10 to about 12 carbon atoms.

In the present invention, preferred isoparaffin include, for example, trimer, tetramer, pentamer, and hexamer of isobutene, and mixtures thereof. Commercially available isoparaffin may have distributions of its polymerization degree, and may be mixtures of, for example, trimer, tetramer, pentamer, and hexamer. What is meant by tetramer herein is that a commercially available isoparaffin hydrocarbons in which tetramer has the highest content, i.e., tetramer is included at a level of preferably 70% or more, more preferably 80% or more, still more preferably 85% or more.

### Mineral oil

The mineral oils useful herein are those having a viscosity of, preferably from about 0.5mm²· s⁻¹ to about 130mm²· s⁻¹, more preferably from about 0.8mm²· s⁻¹ to about 120mm²· s⁻¹, still more preferably from about 1mm²· s⁻¹ to about 100mm²· s⁻¹, even more preferably from about 1.5mm²· s⁻¹ to about 90mm²· s⁻¹, at 37.8°C.

Preferably, the mineral oils useful herein are non-volatile and/or those having from about 18 to about 60 carbon atoms, more preferably from about 20 to about 50 carbon atoms, still more preferably from about 20 to about 40 carbon atoms.

### Volatile Silicone compounds

The volatile silicone compounds useful herein include polyalkyl or polyaryl siloxanes with the following structure (I): 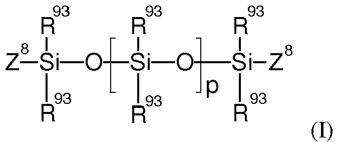 wherein R⁹³ is independently alkyl or aryl, and x is an integer from about 0 to about 5. Z⁸ represents groups which block the ends of the silicone chains. Preferably, R⁹³ groups include methyl, ethyl, propyl, phenyl, methylphenyl and phenylmethyl, Z⁸ groups include hydroxy, methyl, methoxy, ethoxy, propoxy, and aryloxy. More preferably, R⁹³ groups and Z⁸ groups are methyl groups. The preferred volatile silicone compounds are hexamethyldisiloxane, octamethyltrisiloxane, decamethyltetrasiloxane, hexadecamethylheptasiloxane. Commercially available volatile silicone compounds useful herein include octamethyltrisiloxane with tradename SH200C-1cs, decamethyltetrasiloxane with tradename SH200C-1.5cs, hexadecamethylheptasiloxane with tradename SH200C-2cs, all available from Dow Corning.

The volatile silicone compounds useful herein also include a cyclic silicone compound having the formula: 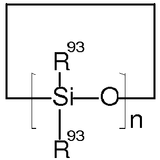 wherein R⁹³ is independently alkyl or aryl, and n is an integer of from 3 to 7. Preferably, R⁹³ groups include methyl, ethyl, propyl, phenyl, methylphenyl and phenylmethyl. More preferably, R⁹³ groups are methyl groups. The preferred volatile silicone compounds are octamethylcyclotetrasiloxane, decamethylcyclopentasiloxane, tetradecamethylcyclohexasiloxane. Commercially available volatile silicone compounds useful herein include octamethylcyclotetrasiloxane with tradename SH244, decamethylcyclopentasiloxane with tradename DC 345 all available from Dow Corning.

### HYDROPHILIC MATERIAL

The composition of the present invention comprises a hydrophilic material. The hydrophilic material is included at a level by weight of the composition of, from about 0.0005% to about 0.1%, preferably from about 0.001% to about 0.07%, more preferably from about 0.03% to about 0.05%.

The hydrophilic materials include, for example, panthenol, panthenyl ethyl ether, water-soluble plant extracts, Vitamin B₃ compounds such as niacinamide, Ascorbic acid compounds such as Ascorbyl glucosid, Peptides, hydrophilic polyols and mixtures thereof. Hydrophilic polyols contain, for example, glycerin diglycerin, propylene glycol, dipropylene glycol, butylene glycol, hexylene glycol, sorbitol, ethoxylated glucose, 1, 2-hexane diol, hexanetriol, erythritol, trehalose, xylitol, maltitol, maltose, glucose, fructose, sodium chondroitin sulfate, sodium hyaluronate, sodium adenosin phosphate, sodium lactate, pyrrolidone carbonate, glucosamine, cyclodextrin, and mixtures thereof. Hydrophilic polyols also include water-soluble alkoxylated nonionic polymers such as polyethylene glycols and polypropylene glycols having a molecular weight of up to about 1000 such as those with CTFA names PEG-200, PEG-400, PEG-600, PEG-1000, and mixtures thereof.

Among the hydrophilic materials, preferred are those selected from the group consisting of panthenol, panthenyl ethyl ether, and mixtures thereof.

### LIQUID FATTY ALCOHOL

The composition of the present invention comprises a liquid fatty alcohol. The liquid fatty alcohol is included at a level by weight of the composition of, from about 0.035% to about 15%, preferably from about 0.1% to about 10%, more preferably from about 0.3% to about 7%. The liquid fatty alcohol is included at a level such that the weight ratio of the hydrophilic material to the liquid fatty alcohol is 1:85 to 1:600, more preferably from about 1:100 to about 1:350, in view of dissolving the hydrophilic material. When the liquid fatty alcohol is included at higher levels than the above ranges, the composition may have at least one of the following tendencies: increased sticky feel, less conditioned feel, undesirable foaming during usage and/or transportation, and reduced stability at lower temperature.

The liquid fatty alcohols useful herein are those having 10 carbon atoms or more, and are liquid at 25°C. Preferably, such liquid fatty alcohols have a melting point of 25°C or lower, more preferably 20°C or lower.

The liquid fatty alcohols useful herein include, for example: lauryl alcohol; a mixture of lauryl alcohol and myristyl alcohol; oleyl alcohol; isocetyl alcohol; isostearyl alcohol; and mixtures thereof. Preferably, the liquid fatty alcohol is selected from the group consisting of: lauryl alcohol; and a mixture of lauryl alcohol and myristyl alcohol, in view of its solubility in the base oil and its solubility of panthenol.

### SILICONE CONDITIONING AGENT

The composition of the present invention can contain a silicone conditioning agent. The silicone conditioning agent can be included at a level by weight of the composition of, preferably from about 0.1%, more preferably from about 0.2%, still more preferably from about 0.5% in view of providing dry conditioning benefit such as reduced friction, and preferably to about 15%, more preferably to about 10%, still more preferably to about 8%, even more preferably to about 5% in view of conditioning benefits and/or product clarity.

The silicone conditioning agents useful herein are non-volatile, and preferably liquid at 25°C. The silicone conditioning agents useful herein have an apparent viscosity of, preferably from about 200 to about 300,000mm ²· s⁻¹, more preferably from about 200 to about 50,000mm²· s⁻¹, at 25°C, in view of providing conditioning benefits.

The silicone conditioning agents useful herein are hydrophobic and those having a HLB of about 8 or less, preferably 5 or less.

The silicone conditioning agents useful herein are, preferably, in non-emulsion form.

### Aminosilicone

In the present invention, the silicone conditioning agent is preferably an aminosilicone. Preferably, aminosilicones useful herein have an amine content of less than about 0.12 m mol/g, more preferably less than about 0.1 m mol/g, still more preferably less than about 0.08m mol/g, even more preferably less than about 0.06m mol/g, in view of friction reduction benefit. It has been surprisingly found that higher levels of nitrogen (amine functional groups) in the amino silicone tend to result in less friction reduction, and consequently less conditioning benefit from the aminosilicone.

Aminosilicone useful herein are those which conform to the general formula (I):

(R₁)ₐG₃₋ₐ-Si-(-OSiG₂)ₙ-(-OSiG_{b}(R₁)_{2-b})ₘ-O-SiG₃₋ₐ(R₁)ₐ

wherein G is hydrogen, phenyl, hydroxy, or C₁-C₈ alkyl, preferably methyl; a is an integer having a value from 1 to 3, preferably 1; b is 0, 1 or 2, preferably 1; n is a number from 1 to 2,000 , preferably from 100 to 1,800, more preferably from 300 to 800, still more preferably 500-600; m is 0; R₁ is a monovalent radical conforming to the general formula CqH_{2q}L, wherein q is an integer having a value from 2 to 8 and L is selected from the following groups: -N(R₂)CH₂-CH₂-N(R₂)₂; -N(R₂)₂; -N(R₂)₃A⁻; -N(R₂)CH₂-CH₂-NR₂H₂A⁻; wherein R₂ is hydrogen, phenyl, benzyl, or a saturated hydrocarbon radical, preferably an alkyl radical from about C₁ to about C₂₀; A is a halide ion. L is preferably -N(CH₃)₂ or -NH₂, more preferably-NH₂.

One highly preferred amino silicones are those corresponding to formula (I) wherein m=0, a=1, q=3, G=methyl, n is preferably from about 1400 to about 1700, more preferably around 1600; and L is -N(CH₃)₂ or -NH₂, more preferably -NH₂. Another further highly preferred amino silicones are those corresponding to formula (I) wherein m=0, a=1, q=3, G=methyl, n is preferably from about 400 to about 800, more preferably about 500 to around 600; and L is -N(CH₃)₂ or -NH₂, more preferably -NH₂.

Such preferred aminosilicones can be called as terminal aminosilicones, as one or both ends of the silicone chain are terminated by nitrogen containing group. It is also surprisingly found that, such terminal aminosilicones provide improved friction reduction compared to graft aminosilicones.

### ADDITIONAL COMPONENTS

The composition of the present invention may include other additional components, which may be selected by the artisan according to the desired characteristics of the final product and which are suitable for rendering the composition more cosmetically or aesthetically acceptable or to provide them with additional usage benefits. Such other additional components generally are used individually at levels of from about 0.001% to about 10%, preferably up to about 5% by weight of the composition.

A wide variety of other additional components can be formulated into the present compositions. These include: other conditioning agents such as hydrolysed collagen with tradename Peptein 2000 available from Hormel, vitamin E with tradename Emix-d available from Eisai, hydrolysed keratin, proteins, and nutrients; preservatives such as benzyl alcohol, methyl paraben, propyl paraben and imidazolidinyl urea; coloring agents, such as any of the FD&C or D&C dyes; perfumes; and antidandruff agents such as zinc pyrithione and salicylic acid.

### PRODUCT FORMS

The hair care compositions of the present invention can be hair conditioning and/or hair styling products, and the like.

The hair care compositions of the present invention are, preferably, in the form of leave-on products. The hair care compositions of the present invention can be formulated in a wide variety of product forms, including gels, sprays, oils, liquid and serum. Preferably, the compositions of the present invention are in a liquid form such as oil, liquid and serum.

### METHOD OF USE

For a leave-on form, the hair care composition is preferably applied to wet or damp hair prior to drying of the hair. After such hair care compositions are applied to the hair, the hair is dried and styled in accordance with the preference of the user. In the alternative, it may be applied to already dry hair, and the hair is then combed or styled, and dried in accordance with the preference of the user.

### EXAMPLES

The following examples further describe and demonstrate embodiments within the scope of the present invention. The examples are given solely for the purpose of illustration and are not to be construed as limitations of the present invention, Where applicable, ingredients are identified by chemical or CTFA name, or otherwise defined below.

**[Compositions]**

| Components | Ex.1 | Ex.2 | Ex.3 | Ex.4 | Ex. i | Ex. ii | Ex.iii |
|---|---|---|---|---|---|---|---|
| Volatile isoparaffin *1 | 50.0 | 50.0 | 50.0 | 50.0 | 50.0 | 50.0 | 50.0 |
| Non-volatile mineral oil-1 *2 | q.s. to 100% | q.s. to 100% | q.s. to 100% | q.s. to 100% | q.s. to 100% | q.s. to 100% | q.s. to 100% |
| Non-volatile mineral oil-2 *3 | 10.0 | - | - | - | - | - | - |
| D-Panthenol | 0.005 | 0.005 | 0.03 | 0.03 | 0.03 | 0.03 | 0.005 |
| Lauryl alcohol | 0.7 | 0.7 | 4.0 | - | 1.0 | 30.0 | - |
| Oleyl alcohol | - | - | - | 7.0 | - | - | - |
| Laureth-3 | - | - | - | - | - | - | 0.7 |
| Aminosilicone *4 | 1.0 | 2.0 | 2.0 | 2.0 | 2.0 | 2.0 | 2.0 |
| Perfume | 0.4 | 0.4 | 0.4 | 0.4 | 0.4 | 0.4 | 0.4 |
| Turbidity (NTU) at 25°C | 0.4 | < 2.0 | < 2.0 | < 2.0 | > 5.0 | < 2.0 | > 5.0 |
| Stickiness/Greasiness | C1 | C1 | C | C1 | - | X | - |

| | | | | | | | |
|---|---|---|---|---|---|---|---|
| Definitions of Components *1 Volatile Isoparaffin: Trimer of isobutene having a viscosity of 1.4 mm²· s⁻¹ at 37.8°C *2 Non-volatile mineral oil-1: Having a viscosity of about 11-14mm²· s⁻¹ at 37.8°C *3 Non-volatile mineral oil-2: Having a viscosity of about 70mm²· s⁻¹ at 37.8°C. *4 Aminosilicone: Terminal aminosilicone which is available from GE having a viscosity of about 1O,000mm²· s⁻¹ at 25°C, and having following formula: (R₁)ₐG₃₋ₐ-Si-(-OSiG₂)ₙ-(-OSiG_{b}(R₁)_{2-b})ₘ-O-SiG₃₋ₐ(R₁)ₐ wherein G is methyl; a is an integer of 1; n is a number from 400 to about 600; m is an integer of 0; R₁ is a monovalent radical conforming to the general formula C_{q}H_{2q}L, wherein q is an integer of 3 and L is -NH₂ | | | | | | | |

### Method of Preparation

The hair conditioning compositions of "Ex. 1" through "Ex. 4" and "Ex. i" through "Ex. iii" as shown above can be prepared by any conventional method well known in the art. They are suitably made as follows:
Hydrophilic materials such as panthenol and liquid fatty alcohols are added to the base oil with agitation at room temperature until homogenized. If included, other components such as silicones and perfumes are added to the mixture with agitation.

### Properties and Conditioning benefits

Turbidity is measured by the method described above. For some of the above compositions, stickiness/greasiness are evaluated by the following methods. The results of the evaluation are shown above.

### Stickiness/Greasiness

Stickiness/Greasiness is evaluated by 6 panelists, when applying 10ml of the composition.
C: Control
C1: Equal to Control
X: From 3 to 6 panelists answered that the composition had sticker/greasier feel compared to Control.

Examples 1 through 4 are hair conditioning compositions of the present invention which are particularly useful for leave-on use. The embodiments disclosed and represented by the previous "Ex. 1" through "Ex. 4" have many advantages. For example, they provide reduced greasy feel while solubilizing hydrophilic components such as panthenol, and have a homogeneous clear product appearance. Such advantages can be understood by the comparison between the examples of the present invention and comparative examples "Ex. i" through "Ex. iii". For example, the compositions of the comparative examples "Ex. i" and "Ex. iii" does not have clear product appearance. For example, the composition of the comparative example "Ex. ii" has increased stickiness/greasiness, compared to the compositions "Ex. 1" through "Ex. 3" of the present invention.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document the meaning or definition assigned to that term in this document shall govern.

While particular embodiments of the present invention have been illustrated and described, it would be obvious to those skilled in the art that various other changes and modifications can be made.

## Claims

1. A non-aqueous clear hair care composition comprising by weight:
from 61% to 99.9% of a base oil consisting of: an isoparaffin, a mineral oil, a volatile silicone compound, and mixtures thereof;
from 0.0005% to 0.1% of an hydrophilic material;
from 0.035% to 15% of a liquid fatty alcohol having 10 carbon atoms or more;
wherein the weight ratio of the hydrophilic material to the liquid fatty alcohol is from 1:85 to 1:600; and
1% or less of water by weight of the composition.

2. The hair care composition of Claim 1, wherein the hydrophilic material is selected from the group consisting of panthenol, panthenyl ethyl ether, and mixtures thereof.

3. The hair care composition of Claim 1, wherein the liquid fatty alcohol is selected from the group consisting of: lauryl alcohol; a mixture of lauryl alcohol and myristyl alcohol; oleyl alcohol; isocetyl alcohol; isostearyl alcohol; and mixtures thereof.

4. The hair care composition of Claim 1, wherein the liquid fatty alcohol is selected from the group consisting of: lauryl alcohol; and a mixture of lauryl alcohol and myristyl alcohol.

5. The hair care composition of Claim 1, wherein the base oil consisting of: an isoparaffin, a mineral oil, and mixtures thereof.

6. The hair care composition of Claim 1 further comprising a non-volatile silicone conditioning agent.

7. The hair care composition of Claim 1 wherein the composition is substantially free of anionic surfactants; cationic surfactants; amphoteric surfactants; zwitterionic surfactants; nonionic surfactants having a HLB of 5 or more; and mixtures thereof.

8. The hair care composition of Claim 1, wherein the composition is for leave-on use.

9. The hair care composition of Claim 1, wherein the composition has a turbidity of 2.0NTU or less at 25°C.

## Patentansprüche

1. Nicht-wässrige Haarpflegezusammensetzung, die, bezogen auf das Gewicht, Folgendes umfasst:
von 61 % bis 99,9 % ein Basisöl, das aus Folgendem besteht: einem Isoparaffin, einem Mineralöl, einer flüchtigen Silikonverbindung und Gemischen daraus;
von 0,0005 % bis 0,1 % ein hydrophiles Material;
von 0,035 % bis 15 % einen flüssigen Fettalkohol mit 10 Kohlenstoffatomen oder mehr;
wobei das Gewichtsverhältnis des hydrophilen Materials zum flüssigen Fettalkohol bei 1:85 bis 1:600 liegt; und
bezogen auf das Gewicht der Zusammensetzung 1 % Wasser oder weniger.

2. Haarpflegezusammensetzung nach Anspruch 1, wobei das hydrophile Material ausgewählt ist aus der Gruppe bestehend aus Panthenol, Panthenylethylether und deren Gemischen.

3. Haarpflegezusammensetzung nach Anspruch 1, wobei der flüssige Fettalkohol ausgewählt ist aus der Gruppe bestehend aus: Laurylalkohol; einem Gemisch aus Laurylalkohol und Myristylalkohol; Oleylalkohol; Isocetylalkohol; Isostearylalkohol; und deren Gemischen.

4. Haarpflegezusammensetzung nach Anspruch 1, wobei der flüssige Fettalkohol ausgewählt ist aus der Gruppe bestehend aus: Laurylalkohol; und einer Mischung aus Laurylalkohol und Myristylalkohol.

5. Haarpflegezusammensetzung nach Anspruch 1, wobei das Basisöl aus Folgendem besteht: einem Isoparaffin, einem Mineralöl und deren Gemischen.

6. Haarpflegezusammensetzung nach Anspruch 1, ferner ein nichtflüchtiges Silikon-Konditioniermittel umfassend.

7. Haarpflegezusammensetzung nach Anspruch 1, wobei die Zusammensetzung im Wesentlichen frei ist von anionischen Tensiden; kationischen Tensiden; amphoteren Tensiden; zwitterionischen Tensiden; nicht-ionischen Tensiden mit einem HLB von 5 oder mehr; und deren Gemischen.

8. Haarpflegezusammensetzung nach Anspruch 1, wobei die Zusammensetzung nicht ausgespült werden muss.

9. Haarpflegezusammensetzung nach Anspruch 1, wobei die Zusammensetzung bei 25 °C eine Trübung von 2,0 NTU oder weniger aufweist.

## Revendications

1. Composition limpide non aqueuse pour soins capillaires comprenant, en poids :
de 61 % à 99,9 % d'une huile de base constituée de : une isoparaffin, une huile minérale, un composé de silicone volatile, et leurs mélanges ;
de 0,0005 % à 0,1 % d'un matériau hydrophile ;
de 0,035 % à 15 % d'un alcool gras liquide comportant 10 atomes de carbone ou plus ;
dans laquelle le rapport pondéral du matériau hydrophile sur l'alcool gras liquide va de 1:85 à 1:600 ; et
1 % ou moins d'eau en poids de la composition.

2. Composition pour soins capillaires selon la revendication 1, dans laquelle le matériau hydrophile est choisi dans le groupe constitué de panthénol, éther éthylique de panthényle, et leurs mélanges.

3. Composition pour soins capillaires selon la revendication 1, dans laquelle l'alcool gras liquide est choisi dans le groupe constitué de : alcool laurylique , un mélange d'alcool laurylique et d'alcool myristylique ; alcool oléylique ; alcool isocétylique ; alcool isostéarylique ; et leurs mélanges.

4. Composition pour soins capillaires selon la revendication 1, dans laquelle l'alcool gras liquide est choisi dans le groupe constitué de : alcool laurylique ; et un mélange d'alcool laurylique et d'alcool myristylique.

5. Composition pour soins capillaires selon la revendication 1, dans laquelle l'huile de base est constituée de : une isoparaffine, une huile minérale, et leurs mélanges.

6. Composition pour soins capillaires selon la revendication 1, comprenant en outre un agent de conditionnement siliconé non volatil.

7. Composition pour soins capillaires selon la revendication 1, où la composition est essentiellement dépourvue d'agents tensioactifs anioniques ; d'agents tensioactifs cationiques ; d'agents tensioactifs amphotères ; d'agents tensioactifs zwittérioniques ; d'agents tensioactifs non ioniques possédant un rapport hydrophile-lipophile de 5 ou plus ; et leurs mélanges.

8. Composition pour soins capillaires selon la revendication 1, où la composition est pour une utilisation à conserver.

9. Composition pour soins capillaires selon la revendication 1, où la composition a une turbidité de 2,0 NTU ou moins à 25 °C.
